# EUROPEAN PATENT SPECIFICATION

(11) **EP 4 187 250 B1**
(45) Date of publication and mention of the grant of the patent: **06.03.2024**
(21) Application number: 22209506.9
(22) Date of filing: 24.11.2022
(51) Int. Cl.: G01N 35/00, G01N 33/543, G01N 35/10

(54) **MULTI-CHANNEL PARALLEL PRETREATMENT DEVICE**
MEHRKANALIGE PARALLELVORBEHANDLUNGSVORRICHTUNG
DISPOSITIF DE PRÉTRAITEMENT PARALLÈLE MULTICANAL

(30) Priority: 25.11.2021 CN 202111409471
(43) Date of publication of application: 31.05.2023
(73) Proprietor: Chengdu Illumaxbio Technology Co., Ltd., Chengdu, Sichuan (CN)
(72) Inventor: CHANG, Le, Chengdu (CN); ZHANG, Xingpeng, Chengdu (CN); WEI, Rundan, Chengdu (CN)
(74) Representative: Bayramoglu et al.

(56) References cited:
- CN-A- 103 992 940
- CN-U- 208 109 848
- CN-U- 211 086 318
- CN-U- 211 086 330

## Description

### TECHNICAL FIELD

The present disclosure relates to the technical field of immunodiagnosis and in particular to a multi-channel parallel pretreatment device.

### BACKGROUND

The existing magnetic particle-based reaction testing system has become one of the most popular means of clinical testing and has been widely used in hospitals at all levels due to its easy automation, fast response speed, and high signal-to-noise ratio (SNR). In this system, the pretreatment system related to an magnetic particle reagent directly affects the stability and accuracy of the testing results.

Currently, the most widely used conventional magnetic particle-based chemiluminescence instruments are large-scale instruments. During magnetic separation, a permanent magnet is loaded on the outside of a cuvette to absorb magnetic beads. Afterward, a pipette is used to suck the waste liquid away from the magnetic beads, then a cleaning solution is injected, and the magnetic beads are re-suspended by a mixing mechanism. The process is repeated three times.

However, the existing cleaning mechanism is complex and bulky and requires a dedicated liquid passage system and mixing mechanism. Thus, it involves various components such as a peristaltic pump, plunger pump, solenoid valve, mixing motor, eccentric mechanism, lifting mechanism, cleaning solution disposal mechanism, and waste liquid disposal mechanism. As a result, the instrument has defects of a complex structure, high cost, a high failure rate, and high maintenance expenses. Currently, there is also a device that can actively adsorb magnetic particles with the cooperation of a magnetic rod and a magnetic rod sleeve and realize the cleaning and separation functions through a separate cleaning solution. However, the device cannot achieve pipetting and cannot solve the problem of sample transfer.
CN103992940A discloses an instrument for extracting a bioactive substance. The instrument comprises a rack, a workbench, a liquid transfer assembly and a separation assembly, wherein the liquid transfer assembly comprises a first mounting plate, a first driving unit, pipettes, piston rods and a first driving component; the first driving unit is used for driving the first mounting plate to move along the height direction of the rack; the pipettes are connected on the first mounting plate; the piston rods are inserted in the pipettes and are hermetically fit with the inner walls of the pipettes; the first driving component is used for driving the piston rods to move up and down relative to the pipettes; the separation assembly comprises a second mounting plate, a second driving unit, casings, magnetic bars and a second driving component; the second driving unit is used for driving the second mounting plate to move along the height direction of the rack; the casings are fixed on the second mounting plate; the second driving component is used for driving the magnetic bars to move up and down relative to the casings; the upper ends of the casings are provided with openings, and the lower ends are blind ends; the magnetic bars are inserted in the casings from openings at the upper ends of the casings by way of clearance fit. The instrument improves the bioactive substance extraction efficiency, and the extracted sample has higher purity.

### SUMMARY

Given the above problems in the prior art, the present disclosure provides a multi-channel parallel pretreatment device for magnetic particle-based reaction testing. The present disclosure greatly reduces the complexity of the magnetic separation and cleaning mechanism, improves the reliability of the magnetic separation mechanism, enables multi-channel simultaneous high-precision pipetting, and achieves low cost.

To achieve the above objective, the present disclosure adopts the following technical solution:
The multi-channel parallel pretreatment device includes a magnetic separation and transfer device configured to perform separation and transfer of a magnetic particle reagent and a first drive device configured to drive the magnetic separation and transfer device to move. The magnetic separation and transfer device includes a second mounting bracket, which is provided therein with an injector chamber. A mounting plate is provided above the top of the injector chamber and is provided with piston rods that are connected to the injector chamber in a movable and sealing manner. The second mounting bracket is further provided thereon with a second drive device, which is configured to drive the mounting plate to linearly reciprocate in a vertical direction.

The injector chamber is provided with loading heads, each of which is configured to load a tip head or a magnetic separation sleeve and has a hollow structure.

The free ends of the piston rods are fixedly connected to magnetic rods that are configured to adsorb magnetic beads in the magnetic particle reagent. The second drive device can drive the magnetic rods on the piston rods to pass through the loading heads.

Further, as a specific implementation of the first drive device, the first drive device includes a first mounting bracket, which is provided thereon with a first motor. An output end of the first motor is provided with a first drive screw. The first mounting bracket is further vertically provided with a first linear guide rail. The first linear guide rail is connected to a slider in a slidable manner. The slider is fixedly connected to the second mounting bracket, and the first drive screw is connected to the slider in a threaded manner.

Further, as a specific implementation of the second drive device, the second drive device includes a second motor provided on the second mounting bracket. An output end of the second motor is provided with a second drive screw, which is connected to the mounting plate in a threaded manner. The injector chamber is vertically and fixedly provided with a second linear guide rail that is connected to the mounting plate in a slidable manner.

Further, to implement multi-channel parallel separation and transfer of the magnetic particle reagent and improve the separation and transfer efficiency of the magnetic particle reagent, multiple piston rods are provided and are evenly spaced in a length direction of the mounting plate, and the free end of each of the piston rods is fixedly connected to one magnetic rod.

Multiple loading heads are provided and are evenly spaced in a length direction of the injector chamber, and the loading heads are fitted with the magnetic rods one by one.

Further, to form negative pressure in the channels of the injector chamber in which the piston rods are connected to facilitate the automatic adsorption of the reagent by the tip heads by controlling the expansion and contraction of the piston rods, the multiple piston rods are connected to the injector chamber in a movable and sealing manner through a sealing ring or a sealing washer.

Further, as a specific implementation of a fit between the loading head and the magnetic separation sleeves as well as the tip heads, the loading heads each load the magnetic separation sleeve and the tip head using an interference fit.

Further, the tip head has a pipetting volume of 5 µl-1,000 µl.

Further, to improve the drive efficiency of the first drive device and the second drive device and speed up the response speed, the first drive screw and the second drive screw are provided with T-shaped external threads.

The present disclosure has the following beneficial effects.
1. By introducing the magnetic rods and the disposable magnetic separation sleeves, passive magnetic separation is changed into active magnetic separation, which greatly improves the separation speed and greatly simplifies the magnetic separation process. Since the magnetic field is closer to the magnetic particles and the magnetic adsorption is performed by reciprocating, the active magnetic separation design has higher separation efficiency and shorter separation time, which is far superior to passive magnetic separation. The multi-channel parallel separation further improves the magnetic particle separation and transfer efficiency. The first drive device and the second drive device realize the reciprocation of the magnetic separation sleeves to complete the mixing of the reaction solution and the re-suspension of the cleaning solution. Therefore, the present disclosure has a simple structure, high reliability, and avoids a liquid splash. The present disclosure realizes a multi-channel parallel pipetting function, which enables precise pipetting of the sample or reagent, thus eliminating the need for a complex pipetting structure. In addition, the present disclosure integrates the functions of pipetting, magnetic adsorption, transfer, mixing, and unloading, which greatly improves the degree of automation.
2. The second drive device drives the piston rods to linearly reciprocate in the vertical direction, and the piston rods drive the magnetic rods to linearly reciprocate inside the loading heads and the injector chamber. The design realizes the automatic separation and loading of the magnetic separation sleeves and the tip heads on the loading heads, which does not require an additional mechanism and has strong practicability. The present disclosure has the advantages of high performance, small volume, and automated pipetting, can be applied to point-of-care testing (POCT) products, and has great potential for expansion.
3. The loading heads and the tip heads are in an interference fit to realize the multi-channel parallel pipetting function. The tip head has a pipetting volume of 5 µl-1,000 µl with high pipetting precision and high consistency. Therefore, the present disclosure has high parallel pipetting efficiency and greatly shortens the sample pretreatment time.
4. The multiple piston rods are connected to the injector chamber in a movable and sealing manner through a sealing ring or a sealing washer. The channels of the injector chamber in which the piston rods are connected form a negative pressure. This design facilitates the automatic adsorption of the reagent by the tip heads by controlling the expansion and contraction of the piston rods, avoids the need for other adsorption devices and a liquid passage, and achieves a simple structure.
5. When it is necessary to mix the reaction solution and re-suspend the cleaning solution, the first drive device and the second drive device drive the loading heads with the tip heads and the magnetic separation sleeves to move upward and downward. The design achieves a simple structure, high mixing efficiency, and avoids liquid splashing that may be caused by eccentric mixing.

### BRIEF DESCRIPTION OF THE DRAWINGS

FIG. 1 is a three-dimensional structural view of a multi-channel parallel pretreatment device according to the present disclosure;
FIG. 2 is an enlarged view of a tip head according to the present disclosure; and
FIG. 3 is an enlarged view of a magnetic separation sleeve according to the present disclosure.

Reference Numerals: 1. second mounting bracket; 2. injector chamber; 3. mounting plate; 4. piston rod; 5. loading head; 6. tip head; 7. magnetic separation sleeve; 8. magnetic rod; 9. first mounting bracket; 10. first motor; 11. first drive screw; 12. first linear guide rail; 13. slider; 14. second motor; 15. second drive screw; and 16. second linear guide rail.

### DETAILED DESCRIPTION OF THE EMBODIMENTS

The specific implementations of the present disclosure are described below to facilitate those skilled in the art to understand the present disclosure, but it should be clear that the present disclosure is not limited to the scope of the specific implementations.

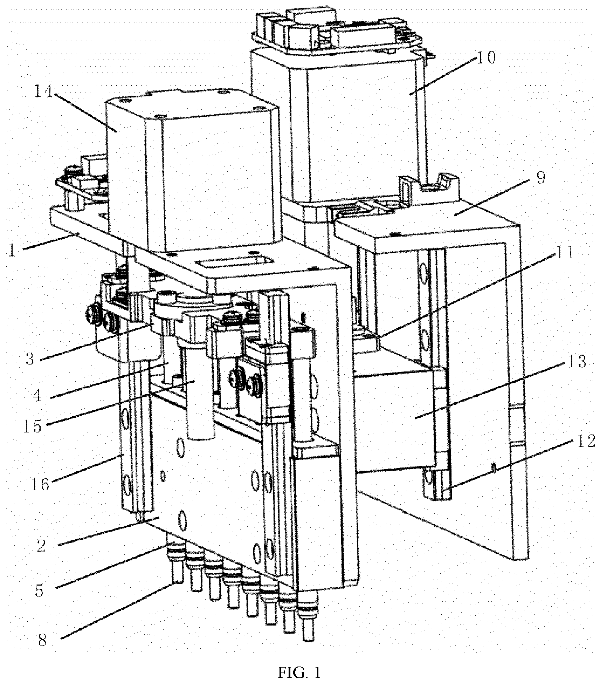

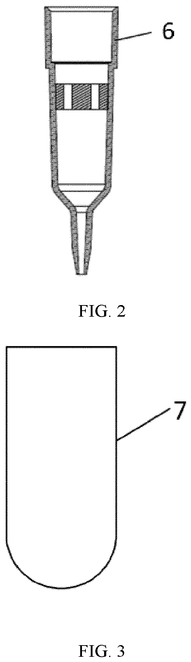

As shown in FIGS. 1 and 2, the present disclosure provides a multi-channel parallel pretreatment device, which includes a magnetic separation and transfer device configured to perform separation and transfer and a first drive device configured to drive the magnetic separation and transfer device to move.

As a specific implementation of the magnetic separation and transfer device, the magnetic separation and transfer device includes second mounting bracket 1. The second mounting bracket 1 is provided therein with injector chamber 2. Mounting plate 3 is provided above the top of the injector chamber 2. The mounting plate 3 is provided with piston rods 4. The piston rods 4 are connected to the injector chamber 2 in a movable and sealing manner. Preferably, but not limited to this, multiple piston rods 4 are connected to the injector chamber 2 in a movable and sealing manner through a sealing ring or a sealing washer.

The second mounting bracket 1 is further provided thereon with a second drive device, which is configured to drive the mounting plate 3 to linearly reciprocate in a vertical direction.

As a specific implementation of the first drive device, the first drive device includes first mounting bracket 9. The first mounting bracket 9 is provided thereon with first motor 10. An output end of the first motor 10 is provided with first drive screw 11. The first mounting bracket 9 is further vertically provided with first linear guide rail 12. The first linear guide rail 12 is connected to slider 13 in a slidable manner. The second mounting bracket 1 is fixedly connected to a slider 13, and the slider 13 is connected to the first drive screw 11 in a threaded manner.

As a specific implementation of the second drive device, the second drive device includes second motor 14 provided on the second mounting bracket 1. An output end of the second motor 14 is provided with second drive screw 15. The second drive screw 15 is connected to the mounting plate 3 in a threaded manner. The injector chamber 2 is provided with a second linear guide rail 16 that is vertical and connected to the mounting plate 3 in a slidable manner.

The injector chamber 2 is provided with loading heads 5 that are configured to load tip heads 6 or magnetic separation sleeves 7. The tip head 6 has a pipetting volume of 5 µl-1,000 µl with high pipetting precision and high consistency.

The loading heads 5 have a hollow structure. The piston rods 4 have free ends that are fixedly connected to magnetic rods 8 that are configured to adsorb magnetic beads in a magnetic particle reagent. The second drive device can drive the magnetic rods 8 on the piston rods 4 to pass through the loading heads 5.

To improve the drive efficiency of the first drive device and the second drive device and speed up the response speed, the first drive screw 11 and the second drive screw 15 are provided with T-shaped external threads.

A magnetic separation process of the multi-channel parallel pretreatment device is as follows. The first motor 10 drives the first drive screw 11 to rotate, and the rotated first drive screw 11 drives the slider 13 to move the second mounting bracket 1 downward. The loading heads 5 of the injector chamber 2 are provided with the magnetic separation sleeves 7. The loading heads 5 and the magnetic separation sleeves 7 are in an interference fit, and the magnetic separation sleeves 7 are loaded by frictional force. When the second mounting bracket 1 is descended to a fixed height, the loading of the magnetic separation sleeves 7 on the loading heads 5 is complete. After the loading of the magnetic separation sleeves 7 is complete, the first motor 10 is controlled to reverse, such that the second mounting bracket 1 is ascended to the highest position. The second motor 14 is rotated to move the mounting plate 3 downward through the second drive screw 15. The downward-moving mounting plate 3 drives the piston rods 4 and the magnetic rods 8 to move downward. When the magnetic rods 8 just reach the bottoms of the magnetic separation sleeves 7, the second motor 14 is stopped. The first motor 10 is controlled to rotate and drive the second mounting bracket 1 to move downward. When the magnetic separation sleeves 7 makes contact with a magnetic particle liquid, the magnetic separation sleeves are descended slowly to reach the bottom of the magnetic particle liquid. In this way, the magnetic particles are gradually captured at the ends of the magnetic separation sleeves 7. Multiple slow ascents or descents may be performed to fully complete the adsorption and separation of the magnetic particles. After the adsorption and separation are completed, the first motor 10 is controlled to drive the second mounting bracket 1 to ascend to the topmost end. All the magnetic particles are adsorbed on the ends of the outer walls of the magnetic separation sleeves 7. The separated liquid is pumped away, and a new cleaning solution is added or a supporting reagent strip is moved, such that the magnetic separation sleeves 7 are above the new cleaning solution. At this time, the magnetic particles need to be suspended in the cleaning solution again for cleaning. The first motor 10 is rotated to cause the second mounting bracket 1 to move down again until the magnetic separation sleeves 7 enter the cleaning solution. The second motor 14 is rotated to drive the piston rods 4, such that the magnetic rods 8 are driven to move up until the magnetic rods 8 are completely separated from the interior of the loading heads 5. The magnetic field disappears, and the magnetic particles are slowly detached from the ends of the magnetic separation sleeves 7. To speed up this process, the first motor can drive the second mounting bracket 1 to reciprocate up and down through different frequencies to ensure that the magnetic particles are fully mixed with the cleaning solution and suspended. After this process is complete, the first motor 10 drives the second mounting bracket 1 to ascend to the highest position again. So far, one cycle of magnetic separation, cleaning, and mixing is complete. If this process is required for more than one time, the above operations are repeated.

The multi-channel parallel pretreatment device can also be used to transfer the magnetic particles into a reagent for mixing, separate the magnetic particles in the reaction solution into the cleaning solution, or transfer the magnetic particles into a substrate solution for reaction and complete luminescence testing. After all separation actions are completed, the magnetic separation sleeves 7 are unloaded. The second motor 14 drives the piston rods 4 and the magnetic rods 8 to move downward. When the magnetic rods 8 reach the bottoms of the magnetic separation sleeves 7, the magnetic rods 8 continue to move downward until all the magnetic separation sleeves 7 are pushed out by the magnetic rods 8 and are separated from the loading heads 5.

A reagent transfer process of the multi-channel parallel pretreatment device is as follows. The first motor 10 is rotated to drive the second mounting bracket 1 to move downward through the slider 13. The second mounting bracket 1 is provided with the loading heads 5 fitted with the tip heads 6. The loading heads 5 and the fitted tip heads 6 are in an interference fit, and the tip heads 6 are loaded by frictional force. When the second mounting bracket 1 is descended to a fixed height, the loading of the fitted tip heads 6 is complete. After the loading is complete, the second mounting bracket 1 is ascended to the highest position. The first motor 10 drives the second mounting bracket 1 to descend to a fixed height. The front ends of the tip heads 6 of the injector chamber 2 with the tip heads 6 are located below the liquid level of the sample or reagent. The second motor 14 is rotated to drive the piston rods 4 to move upward through the mounting plate. Since the injector chamber 2 is a closed chamber, the sample or reagent is sucked into the tip heads 6 by the tip heads 6 under the action of negative pressure. The first motor 10 drives the second mounting bracket 1 to ascend until the tip heads 6 are removed from the liquid level of the sample or reagent. The fitted reagent strip is moved to the desired hole position, and the tip heads 6 are directly above the target hole position. The first motor 10 drives the second mounting bracket 1 to move downward, and the tip heads 6 are driven to run until below the liquid level. The second motor 14 drives the mounting plate 3 to move downward, and the mounting plate 3 drives the piston rods 4 to move downward. Under the action of pressure, the sample or reagent in the cavities of the tip heads 6 is injected into the target hole position. The first motor 10 drives the second mounting bracket 1 to cause the injector chamber 2 to ascend and to separate from the reagent, thereby realizing the automatic transfer of the sample or reagent. Then the piston rods 4 are moved downward, and the magnetic rods 8 are moved downward accordingly. After being moved downward for a fixed distance, the magnetic rods 8 contact filter plugs in the cavities of the fitted tip heads 6. The magnetic rods 8 are continuously moved downward, and the tip heads 6 are separated from the loading heads 5 to realize the unloading of the tip heads 6. If multiple pipetting is required, the tip heads 6 are loaded, and the suction and discharge actions are repeated. The pipetting volume can be 5 µl-1,000 µl. The multi-channel parallel pretreatment device can also be applied for reagent mixing. The tip heads 6 repeatedly sucks and discharges below the liquid level to fully mix the reagent or sample.

When it is necessary to mix the reaction solution and re-suspend the cleaning solution, the first drive device and the second drive device drive the loading heads 5 with the magnetic separation sleeves 7 to move upward and downward. The design achieves a simple structure, high mixing efficiency, and avoids liquid splashing that may be caused by eccentric mixing.

There are multiple piston rods 4. The multiple piston rods 4 are evenly spaced in the length direction of the mounting plate 3. One magnetic rod 8 is fixedly connected to the free end of each piston rod 4. There are multiple loading heads 5. The multiple loading heads 5 are evenly spaced in the length direction of the injector chamber 2. The magnetic rods 8 are fitted with the loading heads 5 one by one. By introducing the magnetic rods 8 and the disposable magnetic separation sleeves 7, passive magnetic separation is changed into active magnetic separation, which greatly improves the separation speed and greatly simplifies the magnetic separation process. Since the magnetic field is closer to the magnetic particles, and the magnetic adsorption is performed by reciprocating, the active magnetic separation design has higher separation efficiency and shorter separation time, which is far superior to passive magnetic separation. The multi-channel parallel separation further improves the magnetic particle separation and transfer efficiency. The first drive device and the second drive device realize the reciprocation of the magnetic separation sleeves 7 to complete the mixing of the reaction solution and the re-suspension of the cleaning solution. Therefore, the present disclosure has a simple structure, high reliability, and avoids a liquid splash. The present disclosure realizes a multi-channel parallel pipetting function, which enables precise pipetting of the sample or reagent, thus eliminating the need for a complex pipetting structure. In addition, the present disclosure integrates the functions of pipetting, magnetic adsorption, transfer, mixing, and unloading, which greatly improves the degree of automation.

The second drive device drives the piston rods 4 to linearly reciprocate in the vertical direction, and the piston rods 4 drive the magnetic rods 8 to linearly reciprocate inside the loading heads 5 and the injector chamber 2. The design realizes the automatic separation and loading of the magnetic separation sleeves 7 and the tip heads 6 on the loading heads 5, which does not require an additional mechanism and has strong practicability. The present disclosure has the advantages of high performance, small volume, and automated pipetting, can be applied to point-of-care testing (POCT) products, and has great potential for expansion.

## Claims

1. A multi-channel parallel pretreatment device, comprising a magnetic separation and transfer device configured to perform separation and transfer of a magnetic particle reagent and a first drive device configured to drive the magnetic separation and transfer device to move, wherein the magnetic separation and transfer device comprises a second mounting bracket (1); a mounting plate (3) provided with piston rods (4); and wherein the second mounting bracket is further provided thereon with a second drive device, which is configured to drive the mounting plate to linearly reciprocate in a vertical direction;
**characterized in that**
the second mounting bracket is provided therein with an injector chamber (2),
the mounting plate is provided above a top of the injector chamber,
the piston rods are connected to the injector chamber in a movable and sealing manner,
the injector chamber is provided with loading heads (5), each of which is configured to load a tip head (6) or a magnetic separation sleeve (7) and has a hollow structure; and
wherein free ends of the piston rods are fixedly connected to magnetic rods (8) that are configured to adsorb magnetic beads in the magnetic particle reagent; and the second drive device is configured to drive the magnetic rods on the piston rods to pass through the loading heads.

2. The multi-channel parallel pretreatment device according to claim 1, wherein the first drive device comprises a first mounting bracket (9), which is provided thereon with a first motor (10); and an output end of the first motor is provided with a first drive screw (11);
the first mounting bracket is further vertically provided with a first linear guide rail (12); the first linear guide rail is connected to a slider in a slidable manner; and the slider is fixedly connected to the second mounting bracket; and
the first drive screw is connected to the slider in a threaded manner.

3. The multi-channel parallel pretreatment device according to claim 2, wherein the second drive device comprises a second motor (14) provided on the second mounting bracket; and an output end of the second motor is provided with a second drive screw (15), which is connected to the mounting plate in a threaded manner; and
the injector chamber is vertically and fixedly provided with a second linear guide rail (16) that is configured to be connected to the mounting plate in a slidable manner.

4. The multi-channel parallel pretreatment device according to claim 1, wherein multiple piston rods are provided and are evenly spaced in a length direction of the mounting plate; and the free end of each of the piston rods is fixedly connected to one magnetic rod; and
multiple loading heads are provided and are evenly spaced in a length direction of the injector chamber; and the loading heads are fitted with the magnetic rods one by one.

5. The multi-channel parallel pretreatment device according to claim 4, wherein the multiple piston rods are connected to the injector chamber in a movable and sealing manner through a sealing ring or a sealing washer.

6. The multi-channel parallel pretreatment device according to claim 4, wherein the loading heads each load the magnetic separation sleeve and the tip head using an interference fit.

7. The multi-channel parallel pretreatment device according to claim 6, wherein the tip head has a pipetting volume of 5 µl-1,000 µl.

8. The multi-channel parallel pretreatment device according to claim 3, wherein the first drive screw and the second drive screw are provided with T-shaped external threads.

## Patentansprüche

1. Mehrkanalige Parallelvorbehandlungsvorrichtung, umfassend eine magnetische Trenn- und Transfervorrichtung, die dazu eingerichtet ist, eine Trennung und einen Transfer eines Magnetpulverreagenzes durchzuführen, und eine erste Antriebsvorrichtung, die dazu eingerichtet ist, die magnetische Trenn- und Transfervorrichtung dazu anzutreiben, sich zu bewegen, wobei
die magnetische Trenn- und Transfervorrichtung umfasst: eine zweite Montagehalterung (1);
und eine Montageplatte (3), die mit Kolbenstangen (4) versehen ist; und wobei
die zweite Montagehalterung ferner mit einer zweiten Antriebsvorrichtung darauf versehen ist, die dazu eingerichtet ist, die Montageplatte dazu anzutreiben, sich linear in einer vertikalen Richtung hin- und herzubewegen; **dadurch gekennzeichnet, dass**
die zweite Montagehalterung mit einer Injektorkammer (2) darin versehen ist,
die Montageplatte über einer Oberseite der Injektorkammer bereitgestellt ist,
die Kolbenstangen beweglich und abgedichtet mit der Injektorkammer verbunden sind,
die Injektorkammer mit Ladeköpfen (5) versehen ist, von denen jeder dazu eingerichtet ist, einen Spitzenkopf (6) oder eine magnetische Trennhülse (7) zu laden, und eine hohle Struktur aufweist; und
wobei freie Enden der Kolbenstangen fest mit magnetischen Stangen (8) verbunden sind, die dazu eingerichtet sind, magnetische Kügelchen in dem Magnetpulverreagenz zu adsorbieren; und die zweite Antriebsvorrichtung dazu eingerichtet ist, die magnetischen Stangen auf den Kolbenstangen dazu anzutreiben, durch die Ladeköpfe zu laufen.

2. Mehrkanalige Parallelvorbehandlungsvorrichtung nach Anspruch 1, wobei die erste Antriebsvorrichtung eine erste Montagehalterung (9) umfasst, die mit einem ersten Motor (10) darauf versehen ist; und ein Ausgangsende des ersten Motors mit einer ersten Antriebsschraube (11) versehen ist;
die erste Montagehalterung ferner in vertikaler Richtung mit einer ersten linearen Führungsschiene (12) versehen ist; die erste lineare Führungsschiene verschiebbar mit einem Schieber verbunden ist; und der Schieber fest mit der zweiten Montagehalterung verbunden ist; und
die erste Antriebsschraube durch eine Gewindeverbindung mit dem Schieber verbunden ist.

3. Mehrkanalige Parallelvorbehandlungsvorrichtung nach Anspruch 2, wobei die zweite Antriebsvorrichtung einen zweiten Motor (14) umfasst, der auf der zweiten Montagehalterung vorgesehen ist; und ein Ausgangsende des zweiten Motors mit einer zweiten Antriebsschraube (15) versehen ist, die mit der Montageplatte durch eine Gewindeverbindung verbunden ist; und
die Injektorkammer in vertikaler Richtung und fest mit einer zweiten linearen Führungsschiene (16) versehen ist, die dazu eingerichtet ist, verschiebbar mit der Montageplatte verbunden zu werden.

4. Mehrkanalige Parallelvorbehandlungsvorrichtung nach Anspruch 1, wobei mehrere Kolbenstangen bereitgestellt sind und in einer Längsrichtung der Montageplatte gleichmäßig beabstandet sind; und das freie Ende jeder der Kolbenstangen fest mit einer magnetischen Stange verbunden ist; und
mehrere Ladeköpfe bereitgestellt sind und in einer Längsrichtung der Injektorkammer gleichmäßig beabstandet sind; und die Ladeköpfe einzeln mit einer jeweiligen der magnetischen Stangen bestückt sind.

5. Mehrkanalige Parallelvorbehandlungsvorrichtung nach Anspruch 4, wobei die mehreren Kolbenstangen durch einen Dichtungsring oder eine Dichtungsscheibe beweglich und abgedichtet mit der Injektorkammer verbunden sind.

6. Mehrkanalige Parallelvorbehandlungsvorrichtung nach Anspruch 4, wobei die Ladeköpfe jeweils die magnetische Trennhülse und den Spitzenkopf unter Verwendung einer Presspassung laden.

7. Mehrkanalige Parallelvorbehandlungsvorrichtung nach Anspruch 6, wobei der Spitzenkopf ein Pipettiervolumen von 5 µl bis 1.000 µl aufweist.

8. Mehrkanalige Parallelvorbehandlungsvorrichtung nach Anspruch 3, wobei die erste Antriebsschraube und die zweite Antriebsschraube mit T-förmigen Außengewinden versehen sind.

## Revendications

1. Dispositif de prétraitement parallèle multicanaux, comprenant un dispositif de séparation et de transfert magnétique configuré pour effectuer la séparation et le transfert d'un réactif particulaire magnétique et un premier dispositif d'entraînement configuré pour entraîner le dispositif de séparation et transfert magnétique à se déplacer, dans lequel
le dispositif de séparation et de transfert magnétique comprend un deuxième support de montage (1) ;
une plaque de montage (3) pourvue de tiges de piston (4) ; et dans lequel
le deuxième support de montage est en outre pourvu sur son dessus d'un deuxième dispositif d'entraînement, lequel est configuré pour entraîner la plaque de montage dans un mouvement de va-et-vient linéaire dans une direction verticale ; **caractérisé en ce que**
le deuxième support de montage est pourvu d'une chambre d'injecteur (2),
la plaque de montage est fournie au-dessus d'une partie supérieure de la chambre d'injecteur,
les tiges de piston sont connectées la chambre d'injecteur d'une façon mobile et étanche,
la chambre d'injecteur est pourvue de têtes de chargement (5), chacune desquelles est configurée pour charger une tête de pointe (6) ou un manchon de séparation magnétique (7) et a une structure creuse ; et
dans lequel les extrémités libres des tiges de piston sont connectées de manière fixe à des tiges magnétiques (8) qui sont configurées pour absorber des billes magnétiques dans le réactif particulaire magnétique ; et le deuxième dispositif d'entraînement est configuré pour entraîner les tiges magnétiques sur les tiges de piston pour passer à travers les tees de chargement.

2. Dispositif de prétraitement parallèle multicanaux selon la revendication 1, dans lequel le premier dispositif d'entraînement comprend un premier support de montage (9), lequel est pourvu sur son dessus d'un premier moteur (10) ; et une extrémité de sortie du premier moteur est pourvue d'une première vis d'entraînement (11) ;
le premier support de montage est en outre pourvu verticalement d'un premier rail de guidage linéaire (12) ; le premier rail de guidage linéaire est connecté à une glissière d'une manière coulissante ; et la glissière est connectée de manière fixe au deuxième support de montage ; et
la première vis d'entraînement est connectée à la glissière d'une manière filetée.

3. Dispositif de prétraitement parallèle multicanaux selon la revendication 2, dans lequel le deuxième dispositif d'entraînement comprend un deuxième moteur (14) fourni sur le deuxième support de montage ; et une extrémité de sortie du deuxième moteur est pourvue d'une deuxième vis d'entraînement (15), laquelle est connectée à la plaque de montage d'une manière filetée, et
la chambre d'injecteur est pourvue verticalement et de manière fixe d'un deuxième rail de guidage linéaire (16) qui est configuré pour être connecté à la plaque de montage d'une manière coulissante.

4. Dispositif de prétraitement parallèle multicanaux selon la revendication 1, dans lequel les tiges de piston sont fournies et sont espacées régulièrement dans une direction longitudinale de la plaque de montage ; et l'extrémité libre de chacune des tiges de piston est connectée de manière fixe à une tige magnétique ; et
plusieurs têtes de chargement sont fournies et sont espacées régulièrement dans une direction longitudinale de la chambre d'injecteur ; et les têtes de chargement sont équipées une à une des tiges magnétiques.

5. Dispositif de prétraitement parallèle multicanaux selon la revendication 4, dans lequel les plusieurs tiges de piston sont connectées à la chambre d'injecteur d'une manière mobile et étanche par le biais d'une bague d'étanchéité ou d'une rondelle d'étanchéité.

6. Dispositif de prétraitement parallèle multicanaux selon la revendication 4, dans lequel les têtes de chargement chargent chacune le manchon de séparation magnétique et la tête de pointe en utilisant un ajustement serré.

7. Dispositif de prétraitement parallèle multicanaux selon la revendication 6, dans lequel la tête de pointe a un volume de pipetage de 5 µl à 1 000 µl.

8. Dispositif de prétraitement parallèle multicanaux selon la revendication 3, dans lequel la première vis d'entraînement et la deuxième vis d'entraînement sont pourvues de filetages extérieurs en forme de T.
